# EUROPEAN PATENT SPECIFICATION

(11) **EP 1 397 165 B1**
(45) Date of publication and mention of the grant of the patent: **18.06.2008**
(21) Application number: 02736350.6
(22) Date of filing: 27.05.2002
(51) Int. Cl.: A61L 2/26

(54) **DISINFECTION CHAMBER AND DEVICE**
DESINFEKTIONSKAMMER UND VORRICHTUNG
ENCEINTE DE DESINFECTION ET DISPOSITIF

(30) Priority: 13.06.2001 SE 0102098
(43) Date of publication of application: 17.03.2004
(73) Proprietor: Getinge Disinfection AB, 351 15 Växjö (SE)
(72) Inventor: RENDELL, Ingvar, S-352 61 Växjö (SE)
(74) Representative: Lindberg, Klas Valter Bo
(86) International application number: PCT/SE2002/001004
(87) International publication number: WO 2002/100447

(56) References cited:
- EP-A1- 0 927 552
- EP-A2- 0 815 874
- EP-A2- 0 815 876
- US-A- 4 900 519
- US-A- 4 915 918
- US-A- 5 183 643

## Description

### Technical Field

The present invention relates to a disinfection chamber for use in a disinfection device for health care goods, such as bed pans, urine bottles and the like, said disinfection device comprises a casing and a door, the chamber in use being intended to receive the goods which are to be disinfected.

This invention further relates to a disinfection device, for disinfection of health care goods, such as bed pans, urine bottles and the like, said device comprising a disinfection chamber, in which said goods are intended to be placed during a disinfection process.

### Technical Background

In health service, disinfection constitutes an important component to avoid spread of infection and growth of bacteria. Currently a great number of different disinfection devices are available on the market, which are intended to be placed e.g. in a hospital ward for disinfection of bed pans, urine bottles etc. A disinfection device is usually made up as follows. The device comprises a disinfection chamber, to which several nozzles extend. The nozzles are connected via, inter alia, a pump to a separate water tank, which is supplied from a public water distribution system. The chamber is traditionally made of a metal material, such as stainless steel.

GB-A-1390018 discloses a bed-pan washing apparatus comprising a steam generating vessel, at least one steam line connecting an upper part of the vessel with the interior of the hopper, a water supply line leading to the vessel, a valve in the water supply line and a water heather in the vessel for generating steam from water supplied thereto by the supply line. The hopper has an outlet at its lower portion. The steam lines are connected to the back of the hopper and a vent pipe is connected to the top of the hopper.

There are however a number of drawbacks of the disinfection chambers currently available. Providing a complete chamber requires, for instance, much edging and welding, which is time-consuming and thus also expensive. The joints and the like formed in connection with the traditional mounting process may also constitute a health risk since bacteria tend to collect in these joints. In order to avoid this, it is possible according to prior-art technique to carefully smooth all joints so as to remove any possible irregularities in which bacteria can grow, but this is also time-consuming. In addition to this, disinfection in this type of chamber tends to require a great deal of energy. Among other things, this is due to the fact that much of the thermal energy supplied is needed to heat the metal chamber material and thus is not used in the disinfection process. Furthermore this thermal energy can be passed on, via the chamber walls, to the casing of the disinfection device and make it hot, which may constitute a risk of a user getting burn injuries and feeling discomfort when touching the casing, unless the disinfection device is provided with a separate insulating layer.

### Object of the Invention

The object of the invention is thus to provide an improved disinfection chamber, in which the above-mentioned drawbacks of prior-art technique are overcome.

### Summary of the Invention

The objects described above are achieved by means of the invention according to claim 1. By making the actual chamber of a polymer material, a number of advantages are obtained. First, plastic material absorbs a very small amount of heat, which means that the energy supplied is used in the disinfection process instead of heating the walls of the chamber. This in turn results in shorter process time as well as lower energy consumption. Moreover, polymer has a natural heat-and sound-insulating property. As a result, it is easy to obtain a disinfection device which is silent, and thus does not have a negative effect on the working environment, at the same time as a cool outer casing is provided without arranging any extra insulation for these purposes. Furthermore, polymer materials have special surface properties, and therefore water droplets do not form to the same extent on a polymer surface as on metal materials. This in turn makes it possible to obtain dry goods faster and using less energy. The forming of droplets can also be negative from the point of view of bacterial growth. Another advantage of the invention is that polymer materials are easy to form into a desirable shape, which makes it easy to optimise the chamber, for instance regarding the chamber volume. In addition, the chamber will be lighter, which among other things facilitates transports and makes them less expensive due to the reduced transport weight. A polymer chamber is also cheap to produce, once a tool has been designed, which facilitates mass production.

Preferably the chamber is made of an injection-moulding material, which makes the chamber very easy to manufacture using prior-art manufacturing methods. The chamber can also comprise a reinforcing material to further improve the self-supporting structure. These reinforcing materials can, for instance, be fibreglass materials with a view to reinforcing the entire chamber, which allows the walls of the chamber to be thinner, thereby saving material and space. The reinforcing material can also be locally situated elements intended to reinforce the chamber, for instance, at fixing points.

According to a preferred embodiment, the injection-moulding polymer material is a propylene plastic material, which is a tested, strong and cheap material. The polymer material can also be mixed with a lime material, such as talc or chalk, in order to obtain stronger and thinner chamber walls.

According to a preferred embodiment, the chamber is formed substantially in one piece. This preferred embodiment has a number of advantages. First, it facilitates the mounting in the disinfection chamber as the fitter only has to mount one part. Moreover, the chamber can be made without joints, which involves a number of advantages. For instance hygienic surfaces are obtained, which reduces the risk of growth of bacterial colonies, which may easily arise in cracks, joints and the like. The absence of joints is also advantageous in terms of wear. Suitably the chamber also has a frame element which is formed integrally with the chamber and intended for mounting of the chamber in the disinfection device. This frame makes it possible to mount the chamber in the disinfection device in a fast and simple and thus also less expensive way. Moreover, a minimum number of joints are provided in the transition between the chamber and a door, which closes the chamber during disinfection, which results in a reduced risk of growth of bacterial colonies.

Suitably the chamber has a shape which is adjusted to the shape of the goods which the device is intended to disinfect. By adjusting the chamber to the shape of the goods, a minimum chamber volume can be obtained, which in turn leads to energy savings since a smaller volume has to be heated. Furthermore, parts such as inlets and outlets are suitably formed integrally with the chamber. This results in advantages since fewer loose components are required, which allows for cheaper manufacture, facilitates mounting in the disinfection device and eliminates the risk of a part being incorrectly mounted, and all that this entails. Finally, the chamber is adapted to be detachably mounted in the disinfection chamber. As a result, the chamber is a replaceable component and thus can easily be replaced if the chamber should be worn or if a chamber of a different shape or with a different nozzle position etc. should be desired.

The above-described purposes of the invention are also achieved by a disinfection device, for disinfection of health care goods, such as bed pans, urine bottles and the like, said device comprising a disinfection chamber, in which said goods are intended to be placed during a disinfection process, said chamber being of the kind as defined above.

### Brief Description of the Drawing

The invention will be described below in more detail in the form of a currently preferred embodiment and with reference to the accompanying drawing.

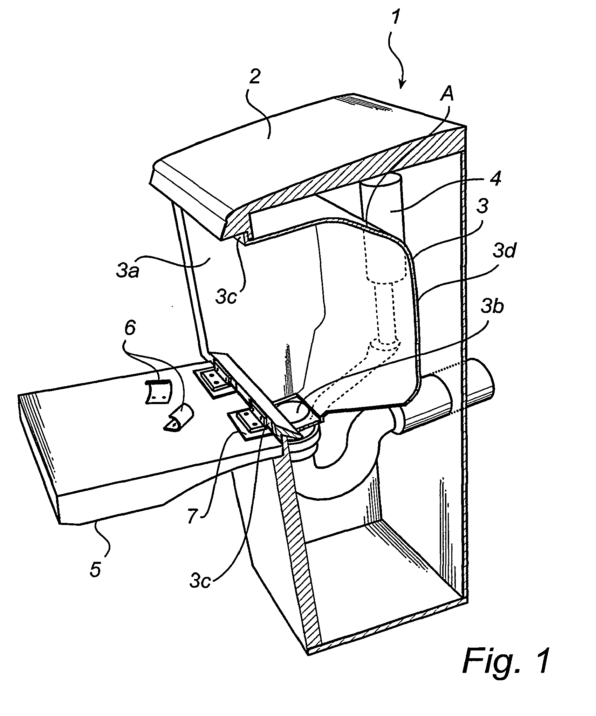

Fig. 1 is a cut-open perspective view of a disinfection device comprising a disinfection chamber according to this invention.

### Description of Preferred Embodiments

A schematic view of a disinfection device 1 for disinfection of bed pans, urine bottles and the like is shown in Fig. 1. The disinfection device 1 comprises essentially a casing 2, which constitutes the outer boundary surface of the disinfection device, and a disinfection chamber 3, which is arranged in said casing 2 and attached to the same. The casing 2 can, for instance, be made of plastic, metal or a combination of these materials. The disinfection device further comprises a water tank 4, which is intended to be filled with water from a public water distribution system and from which water is distributed to a plurality of nozzles (not shown) placed in the chamber 3.

Fig. 1 shows a front-feed disinfection device 1. The chamber 3 has a cup-shaped portion 3d which defines the actual disinfection chamber and has a front opening 3a for introduction of disinfection goods into the chamber and an outlet 3b for discharge of used water, the outlet being placed in the bottom of the chamber in the mounted position of the chamber.

The disinfection device 1 further comprises a door 5, which is movable between a first position, in which it sealingly abuts against the front opening of the chamber to obtain a closed disinfection space defined by the cup-shaped portion 3d and the inside of the door 5, and a second position, in which the front opening of the chamber is uncovered and goods can be introduced into the chamber. Goods holders 6 are suitably arranged at the inside of the door 5. Thus the goods that are to be disinfected can easily be placed in the holder 6 when the door 5 is in the open second position, whereupon the goods are introduced into the chamber when the door is closed, i.e. moved to the first position, whereupon the goods can be disinfected. This construction has the advantage that the goods always reach a correct position in relation to the location of the nozzles, the chamber walls etc. In addition, a user does not have to put his hands into the chamber when introducing or removing goods, which reduces the risk of unintentional contamination.

According to the invention, the chamber 3 is made of a polymer material. Also the door is made of a polymer material. The chamber is formed in one piece, for instance by injection moulding. The chamber further has a number of integrally formed structural parts. These are, for instance, openings for mounting nozzles and a discharge opening. Moreover, the chamber structure comprises an integrally formed mounting frame 3c which extends substantially around the front opening 3a. This frame structure involves a number of advantages compared to prior-art technique, according to which a mounting frame or the like had to be made separately and welded to the chamber, which was expensive and time-consuming, at the same time as the weld joints caused a risk of bacterial growth. By making the entire chamber of a polymer material, it can be made in one piece without any joints, thus eliminating the risk of bacterial growth. The frame 3c has a plurality of fastening openings, through which fastening means, such as screws, can be inserted, after which these are attached to the casing of the disinfection device. This type of attachment also allows the chamber to be attached to the casing in a releasable manner, which makes it possible to replace the chamber when worn or when it is desirable to change the shape etc. of the chamber.

Further advantages of making the chamber of a polymer by injection moulding is that the shape in which the chamber is to be formed when manufactured can easily be adjusted to the goods which are to be disinfected. In the Example shown in Fig. 1, which is primarily intended for disinfection of bed pans, the chamber has for instance been formed with an inclined upper side as shown at A. The thus retracted part of the chamber is not used in disinfection of bed pans and therefore this side of the chamber can be inclined, which results in a smaller chamber volume. Owing to the reduced chamber volume, the amount of energy needed in the disinfection process is also reduced. It is therefore an advantage to "retract" the chamber in parts which are not intended to be used, thus reducing the volume. This is easy to achieve when manufacturing the inventive polymer chamber, but difficult and expensive in traditional chambers.

Furthermore, polymer has a natural sound- and heat-insulating property. Therefore it is easy to provide a disinfection device which is silent, thus not having any negative effect on the working environment, while obtaining a cool outer casing without arranging any extra insulation for this purpose.

The door 5 is in this embodiment fixed to the polymer chamber 3 to the frame 3c by means of hinges or the like. To reinforce the construction at the fixing points, it is possible to arrange or integrate plate-shaped reinforcing elements 7 made, for instance, of metal at these fixing points.

By making the chamber 3 of a polymer material further advantages are obtained. As described above, the chamber can easily comprise an integrated outlet construction, which facilitates the mounting of the chamber in the disinfection device 1. For instance, conduits intended to lead water and/or disinfectant from the respective tanks to the nozzles in the chamber 3 can be formed integrally with the chamber, which further facilitates mounting. It is also possible to easily integrate other parts in the chamber construction, which results in a reduced risk of incorrect mounting, fewer components, reduced mounting time and a construction that is more reliable in operation.

Another advantage of using polymer material is that lime deposits do not form to the same extent on polymer materials as on metal materials.

The polymer material must fulfil the following criteria: Temperature resistance to at least about 90°C, resistance to chemicals present in urine and disinfectants, such as acetates and tensides. Moreover, the material should be relatively flameproof to comply with any fire-protection standards and the like.

A group of materials which has been found to be suitable is thermoplastics, especially polypropylene. Polypropylene has a maximum temperature of about 105°C, it is easy to injection mould/die cast and has good resistance to the above chemicals, and it is resistant to substances with pH values in a wide range, about pH 2-14. In addition, the material is relatively cheap. The forming and the manufacturing of a chamber of this material can be performed according to prior-art technique in the field.

In some applications or in some parts of the chamber, it may be suitable or desirable to reinforce the material. This can be done, for instance, by adding a lime material, such as talc or chalk, or by reinforcing the material, for instance, with fibreglass.

As an alternative, other types of polymers can be used, such as fluoroplastic or PTFE, which is resistant to higher temperatures than propylene plastic, but is on the other hand more expensive and has a greater tendency to shrink. Other polymer materials are also conceivable.

It will be understood that even if the invention is described above with reference to a preferred embodiment, many variants and modifications of the device are feasible without departing from the inventive idea such as defined in the appended claims. The embodiment described herein is, for instance, a front-feed disinfection device, but it will be understood that the inventive chamber is just as useful in other types of disinfection devices, for instance a top-feed disinfection device. Moreover, the chamber can of course be of several different shapes, besides the one shown, to provide an optimal disinfection result in various applications.

## Claims

1. A disinfection chamber (3) for use in a disinfection device (1) for health care goods, such a bed pans, urine bottles and the like, said disinfection device (1) comprises a casing (2) and a door (5), the disinfection chamber being arranged to be attached to the remaining disinfection device (1) and being arranged to partly define in use a disinfection space in said disinfection device (1), the chamber (3) in use being intended to receive the goods which are to be disinfected, **characterised in that** said chamber (3) has a self-supporting structure which is substantially made of a polymer material and that said chamber (3) is adapted to be detachably mounted in said disinfection device (1).

2. A disinfection chamber (3) as claimed in claim 1, wherein said chamber (3) is made of an injection-moulding material.

3. A disinfection chamber (3) as claimed in claim 2, wherein said chamber (3) also comprises a reinforcing material.

4. A disinfection chamber (3) as claimed in claim 2 or 3, wherein said injection-moulding polymer material is a propylene plastic.

5. A disinfection chamber (3) as claimed in claim 1 or 4, wherein said polymer material is mixed with a lime material, such as talc or chalk.

6. A disinfection chamber (3) as claimed in any one of the preceding claims, wherein said chamber (3) is formed substantially in one piece.

7. A disinfection chamber (3) as claimed in claim 6, wherein the chamber (3) has a frame element which is formed integrally with the chamber (3) and is intended for mounting the chamber (3) in said disinfection device (1).

8. A disinfection chamber (3) as claimed in claim 6 or 7, wherein said chamber (3) has a shape which is adjusted to the shape of the goods which the device (1) is intended to disinfect.

9. A disinfection chamber (3) as claimed in claim 1, 6, 7 or 8, wherein parts, such as inlets and outlets, are formed integrally with said chamber.

10. A disinfection device (1), for disinfection of health care goods, such as bed pans, urine bottles and the like, said device (1) comprising a disinfection chamber (3), and a door (5), the disinfection chamber (3) and the door (5) together defining a disinfection space in which said goods are intended to be placed during a disinfection process, **characterised in that** said chamber (3) is of the kind which is defined in any one of claims 1-9.

## Patentansprüche

1. Desinfektionskammer (3) zur Verwendung in einer Desinfektionsvorrichtung (1) für Güter der Krankenpflege wie Bettpfannen, Uringläser und dergleichen, wobei die Desinfektionsvorrichtung (1) ein Gehäuse (2) und eine Tür (3) umfasst und die Desinfektionskammer so eingerichtet ist, dass sie an die übrige Desinfektionsvorrichtung (1) angefügt und so angeordnet wird, dass sie im Gebrauch teilweise einen Desinfektionsraum in der Desinfektionsvorrichtung (1) definiert, und die Kammer (3) im Gebrauch dafür bestimmt ist, die zu desinfizierenden Güter aufzunehmen, **dadurch gekennzeichnet, dass** die Kammer (3) eine selbsttragende Struktur besitzt, die im Wesentlichen aus einem Polymermaterial besteht, und die Kammer (3) so angepasst ist, dass sie abnehmbar in die Desinfektionsvorrichtung (1) eingebaut werden kann.

2. Desinfektionskammer (3) nach Anspruch 1, **dadurch gekennzeichnet, dass** die Kammer (3) aus einem Spritzgussmaterial besteht.

3. Desinfektionskammer (3) nach Anspruch 2, **dadurch gekennzeichnet, dass** die Kammer (3) auch ein Verstärkungsmaterial umfasst.

4. Desinfektionskammer (3) nach Anspruch 2 oder 3, **dadurch gekennzeichnet, dass** das Polymer-Spritzgussmaterial ein Propylenkunststoff ist.

5. Desinfektionskammer (3) nach Anspruch 1 oder 4, **dadurch gekennzeichnet, dass** das Polymermaterial mit einem Kalkmaterial wie Talkum oder Kreide vermischt ist.

6. Desinfektionskammer (3) nach einem der vorangehenden Ansprüche, **dadurch gekennzeichnet, dass** die Kammer (3) im Wesentlichen einteilig ausgebildet ist.

7. Desinfektionskammer (3) nach Anspruch 6, **dadurch gekennzeichnet, dass** die Kammer (3) ein Rahmenelement besitzt, das integral mit der Kammer (3) ausgebildet und zum Einbau der Kammer (3) in die Desinfektionsvorrichtung (1) bestimmt ist.

8. Desinfektionskammer (3) nach Anspruch 6 oder 7, **dadurch gekennzeichnet, dass** die Kammer (3) eine Gestalt besitzt, die der Gestalt der Güter angepasst ist, die durch die Vorrichtung (1) desinfiziert werden sollen.

9. Desinfektionskammer (3) nach Anspruch 1, 6, 7 oder 8, **dadurch gekennzeichnet, dass** Teile wie Zu- und Ausgänge integral mit der Kammer ausgebildet sind.

10. Desinfektionsvorrichtung (1) zur Desinfektion von Gütern der Krankenpflege wie Bettpfannen, Uringläsern und dergleichen, die eine Desinfektionskammer (3) und eine Tür (5) umfasst, wobei die Desinfektionskammer (3) und die Tür (5) zusammen einen Desinfektionsraum definieren, in den die Güter während eines Desinfektionsvorgangs gestellt werden sollen, **dadurch gekennzeichnet, dass** die Kammer (3) von der in einem der Ansprüche 1 bis 9 definierten Art ist.

## Revendications

1. Chambre de désinfection (3) à utiliser dans un dispositif de désinfection (1) pour des produits de soins de santé, tels que des bassins de lits, des récipients de collecte d'urine et autres analogues, ledit dispositif de désinfection (1) comprend une enceinte (2) et une porte (5), la chambre de désinfection étant agencée de sorte à être fixée au reste du dispositif de désinfection (1) et étant agencée afin de partiellement définir pendant l'utilisation, un espace de désinfection dans ledit dispositif de désinfection (1), la chambre (3) lors de son utilisation étant destinée à recevoir les produits qui vont être désinfectés, **caractérisée en ce que** ladite chambre (3) possède une structure autoportante qui est faite essentiellement en un matériau polymère et que ladite chambre (3) est adaptée pour être montée de manière amovible dans ledit dispositif de désinfection (1).

2. Chambre de désinfection (3) telle que revendiquée dans la revendication 1, dans laquelle ladite chambre (3) est faite en un matériau de moulage par injection.

3. Chambre de désinfection (3) telle que revendiquée dans la revendication 2, dans laquelle ladite chambre (3) comprend également un matériau de renforcement.

4. Chambre de désinfection (3) telle que revendiquée dans la revendication 2 ou 3, dans laquelle ledit matériau polymère de moulage par injection est un plastique propylène.

5. Chambre de désinfection (3) telle que revendiquée dans la revendication 1 ou 4, dans laquelle ledit matériau polymère est mélangé avec un matériau à base de chaux, tel que du talc ou de la craie.

6. Chambre de désinfection (3) telle que revendiquée dans l'une quelconque des revendications précédentes, dans laquelle ladite chambre (3) est formée essentiellement en un seul tenant.

7. Chambre de désinfection (3) telle que revendiquée dans la revendication 6, dans laquelle la chambre (3) possède un élément de bâti qui est intégralement formé avec la chambre (3) et est destiné à monter la chambre (3) dans ledit dispositif de désinfection (1).

8. Chambre de désinfection (3) telle que revendiquée dans la revendication 6 ou 7, dans laquelle ladite chambre (3) possède une forme qui est ajustée à la forme des produits que le dispositif est censé désinfecter.

9. Chambre de désinfection (3) telle que revendiquée dans la revendication 1, 6, 7 ou 8, dans laquelle des parties, telles que des entrées et des sorties, sont intégralement formées avec ladite chambre.

10. Dispositif de désinfection (1), pour la désinfection de produits de soins de santé, tels que des bassins de lits, des récipients de collecte d'urine et autres analogues, ledit dispositif (1) comprenant une chambre de désinfection (3) et une porte (5), la chambre de désinfection (3) et la porte (5) définissant conjointement un espace de désinfection dans lequel lesdits produits sont destinés à être placés pendant une procédure de désinfection, **caractérisé en ce que** ladite chambre (3) est du type qui est défini dans l'une quelconque des revendications 1-9.
